# EUROPEAN PATENT SPECIFICATION

(11) **EP 1 131 024 B1**
(45) Date of publication and mention of the grant of the patent: **15.09.2004**
(21) Application number: 99943167.9
(22) Date of filing: 23.09.1999
(51) Int. Cl.: A61F 13/00

(54) **A WOUND DRESSING**
WUNDVERBAND
PANSEMENT

(30) Priority: 28.09.1998 ZA 9808838
(43) Date of publication of application: 12.09.2001
(60) Divisional application: 04014441.2
(73) Proprietor: Vibriant Technology Services Limited, London W1S 4HY (GB)
(72) Inventor: Mouton, Johannes Petrus, Centurion 0157 (ZA)
(74) Representative: Waddington, Richard
(86) International application number: PCT/IB1999/001570
(87) International publication number: WO 2000/018343

(56) References cited:
- EP-A- 0 151 018
- EP-A- 0 849 388
- WO-A-93/22486
- WO-A-96/36304

## Description

THIS INVENTION relates to wound dressings.

According to a first aspect of the invention, there is provided a wound dressing comprising
a first and a second absorbent layer, each absorbent layer being of a non-woven fabric of fibres, and each being able to absorb liquid; and
a screen comprising polyester and cotton fibres between, and bonded to, the two absorbent layers, so that the two absorbent layers and the screen form essentially a single, layered fabric body, the bonding between the first and second absorbent layers and the screen being brought about by a needle-punching process in which the punching density is about 1700 - 1900 punches per square cm.

By "punching density" is meant the number of times a needle perforates the absorbent layers and the screen per square cm in order to bond them together. This is typically conducted on a needleloom which has a needle board with about 30 000 needles and about 6 000 needles per linear metre, at a punching rate of about 300 punches per square cm.

Preferably, the bonding between the first and second absorbent layers may be brought about by a needle-punching process in which the punching density is about 1800 punches per square cm.

The fibres may be porous fibres.

The wound dressing may include at least one liquid permeable layer which is substantially non-adherent to human or animal tissue overlaying, and bonded to, at least one of the absorbent layers.

The wound dressing may include a further liquid permeable layer disposed such that the single, layered fabric body is sandwiched between the two liquid permeable layers.

Each absorbent layer may be of porous polyester fibres. The screen may comprise 80% polyester and 20% cotton fibres. It may have a thread density of about (120 to 150 threads per square inch) 18.6 to 23.2 threads/cm². It may have a yarn count of about 32 to 40. It may have a weight per unit area of approximately 100 g/m².

The absorbent layers of non-woven fabric may be in the form of two fibre batts fabricated on a needleloom, each being made of 100% polyester fibre. The fibre batts may have a fineness of about 1.5 denier and a fibre length of about 7 - 8 cm, preferably about 7,62 cm (i.e. about 3 inches). They may have a weight per unit area of approximately 300 g/m².

The single, layered fabric body may have a mass of about 700 - 750 g/m². It may have a thickness of not more than about 3 mm.

The liquid permeable layer may be of a foraminous or perforated synthetic polymeric material. The synthetic polymeric material may be selected from "MYLAR" (trade name) and "TELFA" (trade name).

The bonding between the or each liquid permeable layer and the or each absorbent layer may be achieved by heat treatment under pressure.

According to another aspect of the invention there is provided a method of making a wound dressing, the method including the steps of
fabricating two needle-punched fibre batts of fibres on a needleloom;
fabricating a tightly woven screen of polyester and cotton fibres;
locating the screen between the needle-punched fibre batts to form a composite structure in which the screen forms an inner layer between the two fibre batts; and
needle-punching the composite structure on a needleloom to produce a single, layered fabric body in a needle-punching process in which the punching density is about 1700 - 1900 punches per square cm.

Preferably, the punching density will be about 1800 punches per square cm.

The method may include the further step of securing a liquid permeable layer, which is substantially non-adherent to human or animal tissue, to at least one side of the layered fabric body.

The needle-punched fibre batts may be of porous polyester. The screen may be fabricated of 80% polyester and 20% cotton yarns. It may be fabricated to have a thread density of about (120 to 150 threads per square inch) 18.6 to 23.2 threads/cm². It may be fabricated to have a yarn count of about 32 to 40. It may be fabricated to have a weight per unit area of approximately 100 g/m².

The composite structure may be needle-punched to produce a single layered fabric body having a mass of about 700 - 750 g/m². The needle punching process may be repeated according to the thickness or effect desired. The composite structure may be needle-punched so that the thickness of the single layered fabric body will typically be not more than about 3 mm.

The fibre batts may have a fineness of about 1.5 denier. They may be fabricated to have a weight per unit area of approximately 300 g/m².

The liquid permeable layer may be of foraminous or perforated synthetic polymeric material. It may be selected from "MYLAR" (trade name) and "TELFA" (trade name).

Securing the liquid permeable layer to the at least one side of the layered fabric body may be achieved by heat treatment under pressure.

The invention extends to a method of removing exudate from a wound, the method including the step of applying to an exudating wound at least one wound dressing as hereinbefore described.

The invention will now be described, by way of example, with reference to the following drawings, in which:
Figure 1 is a schematic, exploded three-dimensional view of a wound dressing in accordance with the invention;
Figure 2 is a schematic, exploded three-dimensional view of another embodiment of a wound dressing in accordance with the invention; and
Figure 3 is a schematic, exploded three-dimensional view of another embodiment of a wound dressing in accordance with the invention.

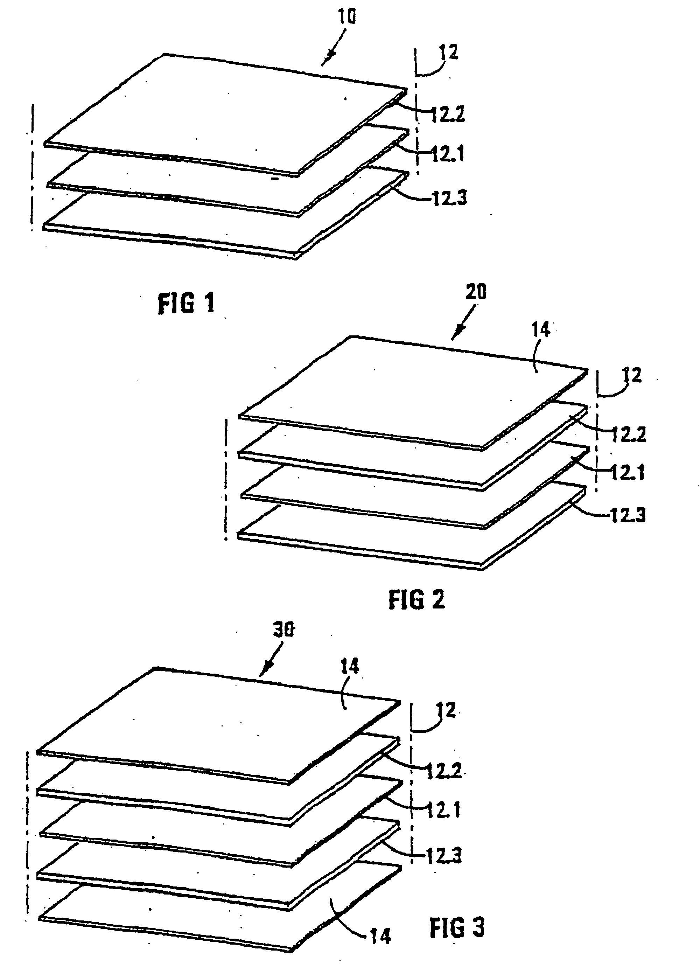

Referring to Figure 1, reference numeral 10 generally indicates a wound dressing in accordance with the invention.

The wound dressing 10 is shown in exploded view in the drawing. The dressing 10 includes a first absorbent layer in the form of a fibre batt 12.2 and a second absorbent layer in the form of a fibre batt 12.3. The fibre batts 12.2 and 12.3 are made of non-woven fabric comprising porous polyester fibres. The dressing 10 includes a tightly woven screen 12.1 comprising polyester and cotton fibres sandwiched between the two fibre batts 12.2 and 12.3. The screen 12.1 is of a woven fabric comprising 80% polyester and 20% cotton yarns and has a thread density of about (120 to 150 threads per square inch) 18.6 to 23.2 threads/cm², a yarn count of about 32 to 40 and a weight of about 100 g/m². The two absorbent layers 12.2 and 12.3 and the screen form essentially a single, layered fabric body, indicated in the drawing by reference numeral 12.

Referring to Figure 2, reference numeral 20 generally indicates another embodiment of a wound dressing in accordance with the invention.

The wound dressing 20 resembles the wound dressing 10 and the same numbers have been used to indicate the same or similar features of the dressings 20 and 10. The dressing 20 differs from the dressing 10 only in that an outer liquid permeable layer 14 is secured to the fibre batt 12.2 so that the wound dressing 20 comprises four separate layers rather than three as in the wound dressing 10.

Referring to Figure 3, reference numeral 30 generally indicates another embodiment of a wound dressing in accordance with the invention. The wound dressing 30 resembles the wound dressing 20 and the same numbers have been used to indicate the same or similar features of the dressings 30 and 20. The dressing 30 differs from the dressing 20 only in that a further outer liquid permeable layer 14 is secured to the fibre batt 12.3 so that the wound dressing 30 comprises five separate layers rather than four as in the wound dressing 20.

The liquid permeable layer 14 is bonded to the composite layer 12 by heat treatment under pressure. In a preferred embodiment of the invention the liquid permeable layers are of the material manufactured by Kendall (Pty) Limited and sold under the name "MYLAR" or the material manufactured by Macmed Healthcare Limited and sold under the trade name "TELFA".

The fibre batts 12.2 and 12.3 are fabricated on a needleloom. Each fibre batt 12.2, 12.3 is made of 100% polyester fibre, with a fineness of about 1.5 denier, a fibre length of about 7,62 cm (about 3 inches) and each has a weight of about 300 g/m². The two fibre batts 12.2 and 12.3 are needle-punched onto each side of the screen 12.1 thus making one completed fibre batt or single, layered fabric body with a middle screen 12.1 and which weighs about 700 g/cm². The thickness of the single, layered fabric body 12 is not more than about 3 mm.

The needle-punching process by which the fibre batts 12.2 and 12.3 and the screen 12.1 are bonded together is conducted on a needleloom which has 30 000 needles on the needle board and 6 000 needles per linear metre. The needle-punching is conducted at a punching rate of 300 punches per square cm so that the resulting punching density is 18 000 punches per square cm. This process pushes fibres from the fibre batts 12.2 and 12.3 through the screen 12.1, thereby bonding the fibre batts 12.2 and 12.3 to the screen 12.1 and to each other.

In different embodiments, the needle-punching process is conducted once or several times, on either side depending on the thickness required. The average thickness of the completed fibre batt is not more than about 3 mm. The Applicant has found that, if the material is much thicker than 3 mm, absorption by the wound dressing is slower and the ability of the wound dressing to "hold and lift" exudate from a wound becomes less effective. Particularly, where a number of layers of the wound dressing of the invention are placed on, or in, a wound which is producing a large amount of fluid, and in which the lower layers become saturated, the Applicant has found that, if the wound dressing is thicker than about 3 mm, transfer of the fluid through successive layers of the wound dressing is less effective. Thus, in use, the wound dressing of the invention may be used in several layers. It may also be folded or rolled for insertion into a wound cavity.

A complete fibre batt with the middle screen as described above was tested by the South African Bureau of Standards (SABS). The test results are tabulated below:

**TABLE 1**

| Tests | Results - SABS samples No. R2455A & R2455B | Requirements as stated in CKS464:1993 (as amended) | Methods of test, CKS 464:1993 (as amended) subclause reference |
|---|---|---|---|
| Construction | The dressing consists of 3 layers and a woven inner layer sandwiched between the 2 outer layers. The dressing has been needle-punched to create adhesion between the 3 layers. | - | Visual Examination |
| Fibre composition, % Outer layers (non-woven fabric) Inner layer (woven fabric) | All polyester Cotton and polyester | Cotton, viscose or thermobonding fibres(e.g. polypropylene) - | 6.5 |
| Mass per unit area, g/m² | 745 | - | 6.6 Determined on the composite sample |
| Bursting strength, kPa | 3 819 | - | 6.7 Determined on the composite sample |
| Sterility | Does not comply | Sterile - packed dressings shall be sterile | 6.15 |
| Fluorescence Non-woven Woven fabric | Occasional spots Fluorescent | Not more than occasional point of intense blue fluorescence | 6.9 |
| Absorption rate, s | 1.1 | 10 max | 6.10 |
| Ash content, g/kg | 0,3 | 5 max | 6.11 |
| pH value of aqueous extract | 7 | 7 ± 2 | BS3266 cold water extraction |
| Freedom from dyes | No yellow, blue or green tint | Percolate may show a yellow colour but not green or blue tint | 6.14 |
| Steam sterilization | Complies | Shall not show any appreciable deterioration in handle | 6.16 |

Being of a different construction, the construction, the mass per unit area and the bursting strength results of samples No. R2455A and R2455B were not compared with the requirements of the above specification.

In Table 1 test samples No. R2455A and R2455B refer to test samples for properties specified in The South African Department of Trade and Industry specification: CKS 464 : 1993 (as amended) for non-woven surgical dressings published by the SABS.

The wound dressing of the invention is a dispersion, high-absorbent, low adherent, disposable wound dressing.

It is an advantage of the invention illustrated that the wound dressing can be used for a wide variety of wounds including high exudating wounds, deep cavity wounds, superficial wounds (with ointments), burn wounds (where the low adherence properties of the wound dressing of the invention are important), transudating wounds, abscesses, ulcers, diabetic foot wounds, external cancer wounds, non-healing wounds, sinus wounds, and the like in both humans and animals. It is a further advantage of the invention illustrated that the wound dressing can be used as a surgical drain. It is a unique feature of the invention that both body fluids and bacteria can be dispersed from, or carried away from, the inside of a wound or cavity by a number of the wound dressings of the invention to the dressings on the outside of the wound or cavity. This enables wounds to heal from the inside and not from the outside. There is no breakdown in the material of the wound dressing after it has made contact with bodily fluids.

Generally, non-adherent absorbent dressings are made of gauze and porous fibres, including natural fibres such as cotton and synthetic fibres such as rayon and combinations thereof. A problem associated with such absorbent dressings is that the porous fibres absorb moisture or fluid from the wound. This leads to a phenomenon known as "strike through". Such wound dressings also to a certain extent, depending upon the type of dressing, tend to adhere to an exudating or transudating wound. Removal of such dressings from the wound often results in reopening of the wound and damage to newly formed granulating tissue.

It is an advantage of the invention illustrated that the tightly woven screen, which forms a discreet layer within the non-woven batt, serves to cause the exudate absorbed by the non-woven fabric part of the dressing to be dispersed along the screen. This serves to prevent or inhibit "strike through" and results in transport of the fluid away from the site of the wound. The advantage of this is that both fluid and bacteria are carried away from the wound thereby encouraging healing of the wound. It is a further advantage of the invention illustrated that the liquid permeable layer does not produce lint, fluff or the like, which are often associated with prior art wound dressings of which the Applicant is aware, and essentially does not adhere to the wound. This reduces both the trauma associated with changing dressings particularly in the case of serious wounds and prevents or inhibits damage to the healing wound caused by the removal of the dressing.

It is a further advantage of the invention illustrated that the three layers comprising the wound dressing can be bonded together by a single needle-punching process. Prior art processes known to the Applicant generally involve more than one needle-punching process to bond different layers together.

Without being bound thereby, the Applicant believes that the needle-punching process by which the wound dressing of the invention is fabricated and which involves a punching density of about 18 00Ø per square cm results in the dispersion action inside the dressing which distinguishes it from prior art absorbent dressings. It is the unique dispersion action inside the dressing which enables the dressing to "hold and lift" exudate or body fluids. Because of the dispersion action of the wound dressing of the invention, even once a dressing is saturated, a successive layer of the wound dressing will cause the fluids to be drawn away from the site of the wound so that healing can take place because of the removal of the fluids and associated bacteria from the wound. The success of the wound dressing of the invention, as a result of the dispersion action of the dressing, appears to be brought about by the choice of fibres and the needle-punching procedure used to fabricate the dressing.

## Claims

1. A wound dressing comprising:
a first and a second absorbent layer, each absorbent layer being of a non-woven fabric of fibres, and each being able to absorb liquid; and
a screen comprising polyester and cotton fibres between, and bonded to, the two absorbent layers so that the two absorbent layers and the screen form essentially a single, layered fabric body, **characterised in that** the screen and the absorbent layers have a needle punched density of about 1700 to 1900 punches per square cm, to thereby bond the screen and the absorbent layers.

2. A wound dressing as claimed in Claim 1, in which the needle punched density is about 1800 punches per square cm.

3. A wound dressing in Claim 1 or Claim 2, in which at least one liquid permeable layer, which is substantially non-adherent to human or animal tissue overlays, and is bonded to, at least one of the absorbent layers.

4. A wound dressing as claimed in any one of the preceding claims, in which each absorbent layer is of porous polyester fibres.

5. A wound dressing as claimed in any one of the preceding claims, in which the screen comprises 80% polyester and 20% cotton fibres.

6. A wound dressing as claimed in any one of the preceding claims, in which the screen has a thread density of about (120 to 150 threads per square inch) 18.6 to 23.2 threads/cm².

7. A wound dressing as claimed in any one of the preceding claims, in which the screen has a yam count of about 32 to 40.

8. A wound dressing as claimed in any one of the preceding claims, in which the screen has a weight per unit area of approximately 100 g/m².

9. A wound dressing as claimed in any one of the preceding claims, in which the absorbent layers of non-woven fabric are in the form of two fibre batts fabricated on a needleloom, each being made of 100% polyester fibre.

10. A wound dressing as claimed in Claim 9, in which the fibre batts have a fineness of about 1.5 denier.

11. A wound dressing as claimed in Claim 10, in which the fibre batts have a weight per unit area of approximately 300 g/m².

12. A wound dressing as claimed in any one of Claims 9 to 11 inclusive, in which the fibre batts have a fibre length of 7 - 8 cm.

13. A wound dressing as claimed in any one of the preceding claims, in which the single, layered fabric body has a mass of about 700 - 750 g/m².

14. A wound dressing as claimed in any one of the preceding claims, in which the single, layered fabric body has a thickness of not more than 3 mm.

15. A wound dressing as claimed in any one of Claims 3 to 14 inclusive, in which the liquid permeable layer is of foraminous or perforated synthetic polymeric material.

16. A wound dressing as claimed in Claim 15, in which the synthetic polymeric material is selected from "MYLAR" (trade name) and "TELFA" (trade name).

17. A wound dressing as claimed in any one of the preceding claims, in which the bonding between the or each liquid permeable layer and the or each absorbent layer is achieved by heat treatment under pressure.

18. A method of making a wound dressing, the method including the steps of
fabricating two needle-punched fibre batts of fibres on a needleloom;
fabricating a tightly woven screen of polyester and cotton fibres;
locating the screen between the needle-punched fibre batts to form a composite structure in which the screen forms an inner layer between the two fibre batts; and
needle-punching the composite structure on a needleloom to produce a single, layered fabric body in a needle-punching process in which the punching density is about 1700 - 1900 punches per square cm.

19. A method as claimed in Claim 18, which includes the further step of securing a liquid permeable layer, which is substantially non-adherent to human or animal tissue, to at least one side of the single layered fabric body.

20. A method as claimed in Claim 18 or Claim 19, in which the needle-punched fibre batts are of polyester.

21. A method as claimed in any one of Claims 18 to 20 inclusive, in which the screen is fabricated of 80% polyester and 20% cotton yarns.

22. A method as claimed in any one of Claims 18 to 21 inclusive, in which the screen is fabricated to have a thread density of about (120 to 150 threads per square inch) 18.6 to 23.3 threads/cm².

23. A method as claimed in any one of Claims 18 to 22 inclusive, in which the screen is fabricated to have a yarn count of about 32 to 40.

24. A method as claimed in any one of Claims 18 to 23 inclusive, in which the screen is fabricated to have a weight per unit area of approximately 100 g/m².

25. A method as claimed in any one of Claims 18 to 24 inclusive, in which the composite structure is needle-punched to produce the single layered fabric body having a mass of 700 - 750 g/m².

26. A method as claimed in Claim 25, in which the composite structure is needle-punched so that the single layered fabric body has a thickness of not more than 3 mm.

27. A method as claimed in any one of Claims 18 to 26 inclusive, in which the fibre batts have a fineness of about 1.5 denier.

28. A method as claimed in any one of Claims 18 to 27 inclusive, in which the fibre batts are fabricated to have a weight per unit area of approximately 300 g/m².

29. A method as claimed in any one of Claims 19 to 28 inclusive, in which the liquid permeable layer is of foraminous or perforated synthetic polymeric material.

30. A method as claimed in any one of Claims 19 to 29 inclusive, in which the synthetic polymeric material is selected from "MYLAR" (trade name) and "TELFA" (trade name).

31. A method as claimed in any one of Claims 19 to 30 inclusive, in which securing the liquid permeable layer to the at least one side of the layered fabric body is achieved by heat treatment under pressure.

## Patentansprüche

1. Wundverband, umfassend:
eine erste und eine zweite Absorptionsschicht, wobei jede Absorptionsschicht aus einem Vliesgewebe aus Fasern hergestellt und in der Lage ist, Flüssigkeit zu absorbieren; und
eine Polyester und Baumwollfasern enthaltende Abschirmung, die zwischen den zwei Absorptionsschichten angeordnet und mit ihnen verbunden ist, derart, dass die zwei Absorptionsschichten und die Abschirmung im Wesentlichen einen einzigen Gewebeschichtkörper bilden, **dadurch gekennzeichnet, dass** die Abschirmung und die Absorptionsschichten eine Nadelstichdichte von ungefähr 1700 bis 1900 Stichen pro cm² aufweisen, um hierdurch die Abschirmung und die Absorptionschichten zu verbinden.

2. Wundverband nach Anspruch 1, bei dem die Nadelstichdichte ungefähr 1800 Stiche pro cm² beträgt.

3. Wundverband nach Anspruch 1 oder 2, bei dem wenigstens eine flüssigkeitsdurchlässige Schicht, die im Wesentlichen nicht an menschlichen oder tierischen Gewebelagen haftet, mit wenigstens einer der Absorptionsschichten verbunden ist.

4. Wundverband nach einem der vorhergehenden Ansprüche, bei dem jede Absorptionsschicht aus porösen Polyesterfasern besteht.

5. Wundverband nach einem der vorhergehenden Ansprüche, bei dem die Abschirmung 80 % Polyester und 20 % Baumwollfasern enthält.

6. Wundverband nach einem der vorhergehenden Ansprüche, bei dem die Abschirmung eine Fadendichte von ungefähr 18,6 bis 23,2 Faden/cm² (120 bis 150 Faden pro Quadratinch) aufweist.

7. Wundverband nach einem der vorhergehenden Ansprüche, bei dem die Abschirmung eine Garnnummer von ungefähr 32 bis 40 aufweist.

8. Wundverband nach einem der vorhergehenden Ansprüche, bei dem die Abschirmung ein Gewicht pro Einheitsfläche von ungefähr 100 g/m² aufweist.

9. Wundverband nach einem der vorhergehenden Ansprüche, bei dem die Absorptionsschichten aus Vliesgewebe in der Form von zwei auf einem Nadelwebstuhl hergestellten Faserpelzen sind, die jeweils aus 100 % Polyesterfasern hergestellt sind.

10. Wundverband nach Anspruch 9, bei dem die Faserpelze eine Feinheit von ungefähr 1,5 Denier haben.

11. Wundverband nach Anspruch 10, bei dem die Faserpelze ein Gewicht pro Einheitsfläche von ungefähr 300 g/m² haben.

12. Wundverband nach einem der Ansprüche 9 bis 11, bei dem die Faserpelze eine Faserlänge von 7 bis 8 cm haben.

13. Wundverband nach einem der vorhergehenden Ansprüche, bei dem der einzige Gewebeschichtkörper eine Masse von ungefähr 700 bis 750 g/m² hat.

14. Wundverband nach einem der vorhergehenden Ansprüche, bei dem der einzige Gewebeschichtkörper eine Dicke von nicht mehr als 3 mm hat.

15. Wundverband nach einem der Ansprüche 3 bis 14, bei dem die flüssigkeitsdurchlässige Schicht aus foraminösem oder perforiertem Kunststoffpolymermaterial gebildet ist.

16. Wundverband nach Anspruch 15, bei dem das Kunststoffpolymermaterial ausgewählt ist aus "MYLAR" (Markenname) und "TELFA" (Markenname).

17. Wundverband nach einem der vorhergehenden Ansprüche, bei dem die Verbindung zwischen der oder jeder flüssigkeitsdurchlässigen Schicht und der oder jeder Absorptionsschicht durch eine Heizbehandlung unter Druck erzielt ist.

18. Verfahren zur Herstellung eines Wundverbands, wobei das Verfahren die Schritte umfasst:
Herstellen von zwei Nadelstich-Faserpelzen aus Fasern auf einem Nadelwebstuhl;
Herstellen einer dicht gewebten Abschirmung aus Polyester und Baumwollfasern;
Anordnen der Abschirmung zwischen den Nadelstich-Faserpelzen zur Bildung einer Verbundstruktur, bei der die Abschirmung eine innere Schicht zwischen den zwei Faserpelzen bildet; und
Nadeln der Verbundstruktur auf einem Nadelwebstuhl zur Herstellung eines einzigen Gewebeschichtkörpers in einem Nadelstichverfahren, bei dem die Stichdichte ungefähr 1700 bis 1900 Stiche pro cm² beträgt.

19. Verfahren nach Anspruch 18, ferner umfassend den weiteren Schritt des Anbringens einer flüssigkeitsdurchlässigen Schicht, die im Wesentlichen nicht an menschlichem oder tierischem Gewebe haftet, an wenigstens einer Seite des einzigen Gewebeschichtkörpers.

20. Verfahren nach Anspruch 18 oder 19, bei dem die Nadelstich-Faserpelze aus Polyester sind.

21. Verfahren nach einem der Ansprüche 18 bis 20, bei dem die Abschirmung aus 80 % Polyester und 20 % Baumwollgamen hergestellt ist.

22. Verfahren nach einem der Ansprüche 18 bis 21, bei dem die Abschirmung derart hergestellt ist, dass sie eine Fadendichte von ungefähr 18,6 bis 23,3 Faden/cm² (120 bis 150 Faden pro Quadratinch) aufweist.

23. Verfahren nach einem der Ansprüche 18 bis 22, bei dem die Abschirmung derart hergestellt ist, dass sie eine Garnnummer von ungefähr 32 bis 40 hat.

24. Verfahren nach einem der Ansprüche 18 bis 23, bei dem die Abschirmung derart hergestellt ist, dass sie ein Gewicht pro Einheitsfläche von ungefähr 100 g/m² aufweist.

25. Verfahren nach einem der Ansprüche 18 bis 24, bei dem die Verbundstruktur genadelt wird, um den einzigen Gewebeschichtkörper mit einer Masse von 700 bis 750 g/m² herzustellen.

26. Verfahren nach Anspruch 25, bei dem die Verbundstruktur derart genadelt wird, dass der einzige Gewebeschichtkörper eine Dicke von nicht mehr als 3 mm aufweist.

27. Verfahren nach einem der Ansprüche 18 bis 26, bei dem die Faserpelze eine Feinheit von ungefähr 1,5 Denier aufweisen.

28. Verfahren nach einem der Ansprüche 18 bis 27, bei dem die Faserpelze derart hergestellt sind, dass sie ein Gewicht pro Einheitsfläche von ungefähr 300 g/m² aufweisen.

29. Verfahren nach einem der Ansprüche 19 bis 28, bei dem die flüssigkeitsdurchlässige Schicht aus foraminösem oder perforiertem Kunststoffpolymermaterial besteht.

30. Verfahren nach einem der Ansprüche 19 bis 29, bei dem das Kunststoffpolymermaterial ausgewählt ist aus "MYLAR" (Markenname) und "TELFA" (Markenname).

31. Verfahren nach einem der Ansprüche 19 bis 30, bei dem eine Anbringung der flüssigkeitsdurchlässigen Schicht an wenigstens einer Seite des Gewebeschichtkörpers durch eine Heizbehandlung unter Druck erzielt wird.

## Revendications

1. Pansement comprenant :
une première et une seconde couches absorbantes, chaque couche absorbante étant en matériau de fibres non tissé, et chacune étant capable d'absorber un liquide ; et
un écran comprenant des fibres de coton et de polyester, entre et liées aux deux couches absorbantes, de sorte que les deux couches absorbantes et l'écran forment essentiellement un corps unitaire de matériau multicouche, **caractérisé en ce que** l'écran et les couches absorbantes ont une densité d'aiguilletage d'environ 1700 à 1900 piqûres par centimètre carré, pour ainsi fixer l'écran et les couches absorbantes.

2. Pansement selon la revendication 1, dans lequel la densité d'aiguilletage est d'environ 1800 piqûres par centimètre carré.

3. Pansement selon la revendication 1 ou la revendication 2, dans lequel au moins une couche perméable aux liquides, laquelle couche est sensiblement non adhérente au tissu animal ou humain, recouvre, et est fixée à au moins l'une des couches absorbantes.

4. Pansement selon l'une quelconque des revendications précédentes, dans lequel chaque couche absorbante est en fibres de polyester poreuses.

5. Pansement selon l'une quelconque des revendications précédentes, dans lequel l'écran comprend 20 % de fibres de coton et 80 % de polyester.

6. Pansement selon l'une quelconque des revendications précédentes, dans lequel l'écran présente une densité de fils d'environ (120 à 150 fils par pouce carré) 18,6 à 23,2 fils/cm².

7. Pansement selon l'une quelconque des revendications précédentes, dans lequel l'écran présente un numéro de fil d'environ 32 à 40.

8. Pansement selon l'une quelconque des revendications précédentes, dans lequel l'écran présente un poids par unité de surface d'environ 100 g/m².

9. Pansement selon l'une quelconque des revendications précédentes, dans lequel les couches absorbantes de matériau non tissé sont sous la forme de deux nappes de fibres fabriquées sur une aiguilleteuse, chacune étant faite de 100 % de fibres de polyester.

10. Pansement selon la revendication 9, dans lequel les nappes de fibres ont une finesse d'environ 1,5 denier.

11. Pansement selon la revendication 10, dans lequel les nappes de fibres ont un poids par unité de surface d'environ 300 g/m².

12. Pansement selon l'une quelconque des revendications 9 à 11 incluses, dans lequel les nappes de fibres ont une longueur de fibre de 7 à 8 cm.

13. Pansement selon l'une quelconque des revendications précédentes, dans lequel le corps unitaire de matériau multicouche présente une masse d'environ 700 à 750 g/m².

14. Pansement selon l'une quelconque des revendications précédentes, dans lequel le corps unitaire de matériau multicouche présente une épaisseur ne dépassant pas 3 mm.

15. Pansement selon l'une quelconque des revendications 3 à 14 incluses, dans lequel la couche perméable aux liquides est en matériau polymère synthétique perforé ou foraminé.

16. Pansement selon la revendication 15, dans lequel le matériau polymère synthétique est choisi parmi le "MYLAR" (nom de marque) et le "TELFA" (nom de marque).

17. Pansement selon l'une quelconque des revendications précédentes, dans lequel la liaison entre la ou chaque couche perméable aux liquides et la ou chaque couche absorbante est réalisée par traitement thermique sous pression.

18. Procédé pour fabriquer un pansement, le procédé comprenant les étapes de :
fabrication de deux nappes de fibres aiguilletées sur une aiguilleteuse ;
fabrication d'un écran tissé de façon serrée de fibres de coton et de polyester ;
positionnement de l'écran entre les nappes de fibres aiguilletées pour former une structure composite dans laquelle l'écran forme une couche interne entre les deux nappes de fibres ; et
aiguilletage de la structure composite sur une aiguilleteuse pour produire un corps unitaire de tissu multicouche dans un procédé d'aiguilletage, dans lequel la densité d'aiguilletage est d'environ 1700 à 1900 perforations par centimètre carré.

19. Procédé selon la revendication 18, qui comprend l'étape supplémentaire de fixer une couche perméable aux liquides, qui est sensiblement non adhérente au tissu animal ou humain, sur au moins un côté du corps unitaire de tissu multicouche.

20. Procédé selon la revendication 18 ou la revendication 19, dans lequel les nappes de fibres aiguilletées sont en polyester.

21. Procédé selon l'une quelconque des revendications 18 à 20 incluses, dans lequel l'écran est fabriqué à partir de 20 % de fils de coton et de 80 % de polyester.

22. Procédé selon l'une quelconque des revendications 18 à 21 incluses, dans lequel l'écran est fabriqué pour avoir une densité de fils d'environ (120 à 150 fils par pouce carré) 18,6 à 23,3 fils/cm².

23. Procédé selon l'une quelconque des revendications 18 à 22 incluses, dans lequel l'écran est fabriqué pour avoir un numéro de fil d'environ 32 à 40.

24. Procédé selon l'une quelconque des revendications 18 à 23 incluses, dans lequel l'écran est fabriqué pour avoir un poids par unité de surface d'environ 100 g/m².

25. Procédé selon l'une quelconque des revendications 18 à 24 incluses, dans lequel la structure composite est aiguilletée pour produire le corps unitaire de tissu multicouche ayant une masse de 700 à 750 g/m².

26. Procédé selon la revendication 25, dans lequel la structure composite est aiguilletée de sorte que le corps unitaire de tissu multicouche présente une épaisseur ne dépassant pas 3 mm.

27. Procédé selon l'une quelconque des revendications 18 à 26 incluses, dans lequel les nappes de fibres ont une finesse d'environ 1,5 denier.

28. Procédé selon l'une quelconque des revendications 18 à 27 incluses, dans lequel les nappes de fibres sont fabriquées pour avoir un poids par unité de surface d'environ 300 g/m².

29. Procédé selon l'une quelconque des revendications 19 à 28 incluses, dans lequel la couche perméable aux liquides est en matériau polymère synthétique perforé ou foraminé.

30. Procédé selon l'une quelconque des revendications 19 à 29 incluses, dans lequel le matériau polymère synthétique est sélectionné parmi le "MYLAR" (nom de marque) et le "TELFA" (nom de marque).

31. Procédé selon l'une quelconque des revendications 19 à 30 incluses, dans lequel la fixation de la couche perméable aux liquides sur au moins un côté du corps de tissu multicouche est réalisée par traitement thermique sous pression.
